# EUROPEAN PATENT APPLICATION

(11) **EP 2 127 681 A1**
(43) Date of publication of application: **02.12.2009**
(21) Application number: 08711312.2
(22) Date of filing: 15.02.2008
(51) Int. Cl.: A61K 48/00, A61K 31/713, A61K 45/00, A61K 47/42, A61P 1/02, A61P 19/08, A61P 29/00

(54) **THERAPEUTIC AGENT FOR PERIODONTAL DISEASE AND ALVEOLAR BONE LOSS DUE TO SURGERY**

(30) Priority: 16.02.2007 JP 2007036135
(71) Applicant: AnGes MG, Inc., Ibaraki-shi, Osaka 5670085 (JP)
(72) Inventor: MORISHITA, Ryuichi, Suita-shi Osaka 565-0871 (JP); NAKAGAMI, Hironori, Suita-shi Osaka 565-0871 (JP); SHIMIZU, Hideo, Suita-shi Osaka 565-0871 (JP)
(74) Representative: Wichmann, Hendrik
(86) International application number: PCT/JP2008/052482
(87) International publication number: WO 2008/099906

(57) **Abstract**

By suppressing transcription activation by NFκB, the present invention was demonstrated to suppress alveolar bone resorption in periodontal disease models, and promote restoration of alveolar bone in periodontal disease-caused bone defect models. Therefore, the present invention provides agents for treating, preventing, and improving periodontal diseases and alveolar bone defects caused by surgical operations, said agents comprising as an active ingredient, a NFκB decoy or such that suppresses the transcription activity of NFκB.

SEQUENCE LISTING

<110>  ANGES MG, INC.

<120>  Therapeutic agent for periodontal disease and alveolar bone
       loss due to surgery

<130>  S66564PCEP

<150>  JP 2007-036135
<151>  2007-02-16

<160>  11

<170>  PatentIn version 3.5

<210>  1
<211>  20
<212>  DNA
<213>  Artificial

<220>
<223>  An artificially synthesized oligonucleotide

<400>  1
ccttgaaggg atttccctcc                                                   20


<210>  2
<211>  10
<212>  DNA
<213>  Artificial

<220>
<223>  An artificially synthesized nucleotide sequence

<400>  2
gggrhtyyhc                                                              10


<210>  3
<211>  10
<212>  DNA
<213>  Artificial

<220>
<223>  An artificially synthesized nucleotide sequence

<400>  3
gggatttccc                                                              10


<210>  4
<211>  10
<212>  DNA
<213>  Artificial

<220>
<223>  An artificially synthesized nucleotide sequence

<400>  4
gggactttcc                                                              10


<210>  5
<211>  20
<212>  DNA
<213>  Artificial

<220>
<223>  An artificially synthesized oligonucleotide

<400>  5
ccttgaaggg atttccctcc                                                    20


<210>  6
<211>  20
<212>  DNA
<213>  Artificial

<220>
<223>  An artificially synthesized oligonucleotide

<400>  6
ggagggaaat cccttcaagg                                                    20


<210>  7
<211>  19
<212>  DNA
<213>  Artificial

<220>
<223>  An artificially synthesized oligonucleotide

<400>  7
agttgaggac tttccaggc                                                     19


<210>  8
<211>  19
<212>  DNA
<213>  Artificial

<220>
<223>  An artificially synthesized oligonucleotide

<400>  8
gcctggaaag tcctcaact                                                     19


<210>  9
<211>  22
<212>  DNA
<213>  Artificial

<220>
<223>  An artificially synthesized oligonucleotide

<400>  9
agttgagggg actttcccag gc                                                 22


<210>  10
<211>  22
<212>  DNA
<213>  Artificial

<220>
<223>  An artificially synthesized oligonucleotide

<400>  10
gcctgggaaa gtcccctcaa ct                                                 22


<210>  11
<211>  20

<212>  DNA
<213>  Artificial

<220>
<223>  An artificially synthesized oligonucleotide

<400>  11
catgtcgtca ctgcgctcat                                                   20

## Description

### Technical Field

The present invention relates to novel therapeutic agents and therapeutic methods for treating periodontal diseases, especially periodontal infections, gingival inflammations, dental periostitis, alveolar pyorrhea, and the like. A therapeutic, preventive, or improving agent according to the present invention is useful against reduction and destruction and reduction of alveolar bone caused by periodontal diseases, alveolar bone defects caused by apical lesions arising from dental caries, and alveolar bone defects caused by surgical operations.

### Background Art

Periodontal infections and gingival inflammations are known to be major periodontal diseases. Both are chronic inflammatory diseases, and as they progress, the periodontal tissue is destroyed, the alveolar bone is reduced due to bone resorption, thus causing loss of tooth support in some cases. In addition, the alveolar bone may become defective due to perforations caused by surgical treatments or apical lesions from progressed dental caries. Therapeutic methods for such periodontal diseases are still being developed.

NFκB (nuclear factor kappa B) is the generic term for a group of transcription factors such as cytokines and adhesion factors, which have the role of regulating expression of genes related to immune reactions. When NFκB binds to binding sites of genomic genes, immune-reaction related genes are overexpressed. Therefore, NFκB is known to be involved with allergic diseases such as atopic dermatitis and rheumatoid arthritis, and autoimmune disorders, which are caused by immune reactions, and with various diseases including ischemic diseases such as cardiac infarction and arterial sclerosis. Administration of a decoy (a decoy-type medicine) against NFκB is known in order to reduce the activity of the subject transcription factor and to treat or prevent diseases caused by the transcription factor (see, for example, Patent Documents 1 and 2). Furthermore, it is known that NFκB decoys are useful in preventing or treating diseases caused by the breakdown of balance between bone formation and bone resorption, especially osteoporosis, through their inhibitory action on the differentiation of bone-marrow cells to osteoclasts (Patent Document 3).

Periodontal infections and gingival inflammations are inflammatory reactions in which inflammatory cytokines are mobilized in the affected areas, and in which activation of NFκB is considered to be involved (Non-patent Documents 1 and 2). Non-patent Document 1 describes that in *in vitro* cultures of healthy human gingival fibroblasts, inflammatory cytokines such as interleukin (IL)-6, IL-8, and monocyte chemotactic protein (MCP)-1 are induced by lipopolysaccharides (LPS), that this induction of inflammatory cytokines by LPS is suppressed by N-arachidonoylethanolamide (anandamide: AEA), and further that although activation of NFκB is induced by LPS, the activation of NFκB is also suppressed by AEA. However, in tissues of patients with some form of periodontal disease, it has only been confirmed that expression of cannabinoid receptor (CB)-1 and CB-2, which are AEA receptors, is increased in human gingival fibroblasts collected from patients with gingival inflammations or periodontal infections compared to healthy human gingival fibroblasts. Accordingly, it is unclear from this document whether AEA has an inflammation-suppressing effect in tissues with some form of periodontal disease, and also it is much less clear whether symptoms of periodontal diseases can be relieved by suppressing NFκB activation.

Non-patent Document 2 describes that compared to healthy gingival tissues, a more activated form of NFκB and less IκB, which is an inhibitory molecule for NFκB, is present in affected gingival tissues of patients with chronic periodontal diseases. However, it does not disclose the association of NFκB activation with the onset and progress of periodontal infections. In addition, it does not indicate the healing of periodontal infections by suppressing NFκB activation.

Accordingly, the above Non-patent Documents do not suggest the recovery from bone loss in periodontal diseases or periodontal infections, and no effective means for recovery have been established.
Patent Document 1: WO 96/35430
Patent Document 2: WO 03/063911
Patent Document 3: WO 2006/064886
Non-patent Document 1: FEBS Letters 580 (2006) 613-619
Non-patent Document 2: J. Periodontol 76 (2005) 1148-1153

### Disclosure of the Invention

### [Problems to be Solved by the Invention]

The present invention aims at providing medicines or methods for treating, preventing, or improving periodontal diseases. Also, the present invention aims at providing medicines or methods for treating, preventing, or improving alveolar bone defects.

### [Means for Solving the Problems]

The present invention showed that administration of NFκB decoys suppresses resorption of alveolar bone in a periodontal disease model, and promotes restoration of alveolar bone in a periodontal disease bone defect model. As a result, the present inventors discovered that NFκB decoys are useful as agents for treating, preventing, and improving periodontal diseases and alveolar bone defects caused by surgical operations and the like. Specifically, the present invention relates to the following:
[1] an agent for treating, preventing, or improving either or both of a periodontal disease and a surgical operation-induced alveolar bone defect, comprising a NFκB decoy as an active ingredient;
[2] the therapeutic, preventive, or improving agent according to [1], wherein the periodontal disease is selected from the group consisting of periodontal infection, gingival inflammation, dental periostitis, and apical lesion arising from dental caries;
[3] the therapeutic, preventive, or improving agent according to [1] or [2], which is an injection;
[4] the therapeutic, preventive, or improving agent according to any one of [1] to [3], which is in a dosage form in which the NFκB decoy is attached to a collagen base material;
[5] the therapeutic, preventive, or improving agent according to any one of [1] to [4], wherein at least one of the linkages between nucleotides comprised in the NFκB decoy is a phosphorothioate linkage;
[6] the therapeutic, preventive, or improving agent according to [5], wherein all of the linkages between nucleotides comprised in the NFκB decoy are phosphorothioate linkages;
[7] the therapeutic, preventive, or improving agent according to any one of [1] to [6], wherein the NFκB decoy is a double-stranded oligonucleotide which is a double strand formed by an oligonucleotide comprising CCTTGAAGGGATTTCCCTCC (SEQ ID NO: 1) and an oligonucleotide comprising a sequence completely complementary thereto;
[8] a method for treating, preventing, or improving either or both of a periodontal disease and a surgical operation-induced alveolar bone defect, comprising the step of administering an NFκB decoy to a subject affected by either or both of a periodontal disease and a surgical operation-induced alveolar bone defect;
[9] the method according to [8], wherein the periodontal disease is selected from the group consisting of periodontal infection, gingival inflammation, dental periostitis, and apical lesion arising from dental caries;
[10] the method according to [8] or [9], wherein the NFκB decoy is administered by injection;
[11] the method according to any one of [8] to [10], which comprises a step of administering a NFκB decoy attached to a collagen base material;
[12] the method according to any one of [8] to [11], wherein at least one of the linkages between nucleotides comprised in the NFκB decoy is a phosphorothioate linkage;
[13] the method according to [12], wherein all of the linkages between nucleotides comprised in the NFκB decoy are phosphorothioate linkages;
[14] the method according to any one of [8] to [13], wherein the NFκB decoy is a double-stranded oligonucleotide which is a double strand formed by an oligonucleotide comprising CCTTGAAGGGATTTCCCTCC (SEQ ID NO: 1) and an oligonucleotide comprising a sequence completely complementary thereto;
[15] use of an NFκB decoy for the manufacture of a pharmaceutical agent for treating, preventing, or improving either or both of a periodontal disease and a surgical operation-induced alveolar bone defect;
[16] the use according to [15], wherein the periodontal disease is selected from the group consisting of periodontal infection, gingival inflammation, dental periostitis, and apical lesion arising from dental caries;
[17] the use according to [15] or [16], which is an injection;
[18] the use according to any one of [15] to [17], which is in a dosage form in which NFκB decoy is attached to a collagen base material;
[19] the use according to any one of [15] to [18], wherein at least one of the linkages between nucleotides comprised in the NFκB decoy is a phosphorothioate linkage;
[20] the use according to [19], wherein all of the linkages between nucleotides comprised in the NFκB decoy are phosphorothioate linkages; and
[21] the use according to any one of [15] to [20], wherein the NFκB decoy is a double-stranded oligonucleotide which is a double strand formed by an oligonucleotide comprising CCTTGAAGGGATTTCCCTCC (SEQ ID NO: 1) and an oligonucleotide comprising a sequence completely complementary thereto.

Alternatively, the present invention provides NFκB decoys for treating, preventing, or improving either or both of periodontal diseases or alveolar bone defects caused by surgical operations. The NFκB decoys according to the present invention is particularly NFκB decoys for treating, preventing, or improving either or both of periodontal diseases or alveolar bone defects caused by surgical operations, and comprises NFκB decoys for topical administration by injection.

### Brief Description of the Drawings

Fig. 1 shows schematic diagrams of a prepared "thread model". The left diagram schematically shows the appearance at the time the model was prepared and the right diagram schematically shows how the alveolar bone is resorbed and regression occurs with time.
Fig. 2 presents a photograph of a sample (mandible) excised one month after model preparation and in which the attached soft tissue is removed with sodium hypochlorite to make the exposed area of the dental roots more visible. The NFκB decoy administered site is indicated by an arrow. The part between the two dashed lines is the exposed area of the dental roots.
Fig. 3 presents photographs showing dental root lengths of maxillary and mandibular lateral incisors (right and left) measured one month after model preparation.
Fig. 4 presents graphs showing the effects of administering the NFκB decoy and scrambled decoy to exposed dental root lengths of maxillary and mandibular lateral incisors (right and left) measured one month, two months, and three months after model preparation.
Fig. 5 is an X-ray picture of the maxilla taken during dissection two months after model preparation. The NFκB decoy administered site is indicated by an arrow. The part between the two dashed lines is the exposed area of the dental roots.
Fig. 6 is an X-ray picture of the mandible taken during dissection two months after model preparation. The NFκB decoy administered site is indicated by an arrow. The part between the two dashed lines is the exposed area of the dental roots.
Fig. 7 is a drawing schematically showing which part of the model's jaw is measured to obtain the length of alveolar bone and length of dental root measured from the dental neck, which are plotted in the graphs of Fig. 8.
Fig. 8 presents graphs plotting the length of remaining alveolar bone/length of dental root ratios obtained from maxillary and mandibular X-ray pictures taken one month, two months, and three months after model preparation. The alveolar bone-preserving effect of NFκB decoy administration is shown.
Fig. 9 presents photographs showing analysis images obtained with DEXA. The measured parts are surrounded by white rectangles, which are the mesial side of the lateral incisor in maxilla and the distal side of the lateral incisor in mandible.
Fig. 10 presents graphs showing DEXA analysis results of the effects of the decoy on bone density changes over time in the thread model.
Fig. 11 is a graph showing amounts of IL-6 in gingival crevicular fluid measured two months after preparation of the thread model.
Fig. 12 shows X-ray pictures of premolars in the periodontal disease bone defect model administered with the NFκB decoy and scrambled decoy taken immediately after operation, and two weeks and four weeks after preparation of the model.
Fig. 13 presents photographs showing cross-sectional views of jaw bone in a periodontal disease bone defect model administered with NFκB decoy and scrambled decoy taken one month after operation. The thickness of cortical bone was measured at the lined parts in the right-hand photograph.
Fig. 14 shows photographs comparing the bone defect sites in a periodontal disease bone defect model administered with NFκB decoy and scrambled decoy one month after operation. Arrows indicate the site administered with scrambled decoy (the left side) and NFκB decoy (the right side), respectively.
Fig. 15 presents, on the left side, an X-ray picture showing the bone defect site where bone density measurement with DEXA was carried out to numerically evaluate the effects of administering NFκB decoy and scrambled decoy on restoration of the bone defect site in a periodontal disease bone defect model. The part surrounded by the circle is the bone defect site. The right side is the analysis image obtained with DEXA. The part surrounded by the rectangle is the bone defect site.
Fig. 16 shows graphs plotted with values obtained through bone density measurement with DEXA at the bone defect site in a periodontal disease bone defect model administered with NFκB decoy and scrambled decoy.

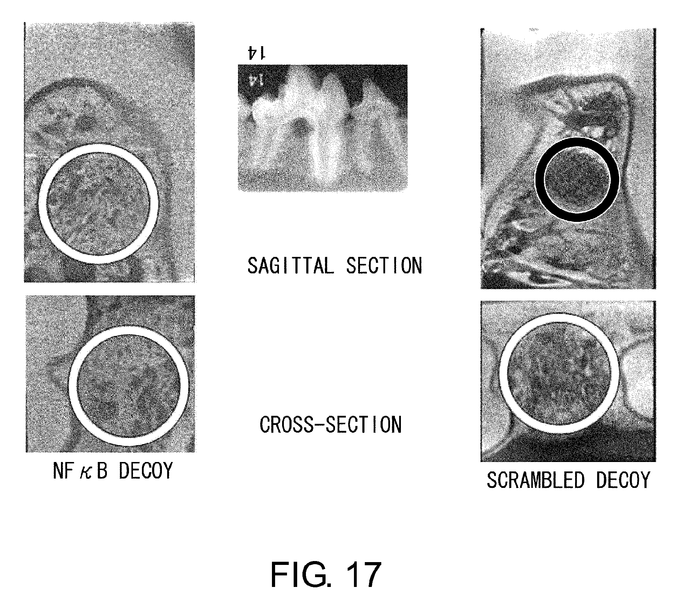

Fig. 17 presents photographs showing CT images of the sites corresponding to bone defect sites one month after operation in a periodontal disease bone defect model administered with NFκB decoy and scrambled decoy. The X-ray picture in the middle was taken to identify the sites corresponding to bone defect sites to be subjected to X-ray micro-CT. The two photographs on the left side are CT images of the bone defect site administered with NFκB decoy, and the two photographs on the right side are CT images of the bone defect site administered with scrambled decoy. In these images, spongy bone parts with the same area are surrounded by circles. The magnification of the X-ray picture in the middle is x2, and in the four CT images on the left and right, the diameter of the outer circle is equivalent to 4 mm.
Fig. 18 presents photographs showing CT images of sites corresponding to bone defect sites two months after operation in a periodontal disease bone defect model administered with NFκB decoy and scrambled decoy. Two photographs on the left are CT images of the bone defect site administered with NFκB decoy, and two photographs on the right are CT images of the bone defect site administered with scrambled decoy. In these images, spongy bone parts with the same area are surrounded by circles. The diameter of the outer circle is equivalent to 4 mm.
Fig. 19 is a graph plotted with the volume ratios oftrabeculae/measured space one month, two months, and three months after operation in a periodontal disease bone defect model administered with NFκB decoy and scrambled decoy.

### Best Mode for Carrying Out the Invention

In the present specification, the term "periodontal diseases" is used as a generic term for lesions in periodontal tissue comprising periodontal infections, gingival inflammations, dental periostitis, alveolar pyorrhea, and reduction and destruction of alveolar bone caused by the above inflammatory lesions, and alveolar bone defects caused by apical lesions arising from dental caries, and the like. In addition, when dental caries progress, the alveolar bone can become defected due to destruction by apical lesions; such symptoms are included in the "periodontal diseases" in the present specification.

Herein, "periodontal infections" comprise any periodontal infections, such as apical periodontitis, juvenile periodontitis, periodontitis simplex, and periodontitis complex, as well as inflammations caused by wounds resulting from any surgical operation in the jaw and periodontal area including oral surgeries for tooth extractions, treatment of tumors and deformities (for example, jaw deformity), reconstructions, implants, and orthodontic treatment. In general, when periodontal infections progress, they cause reduction or destruction of alveolar bone. The term "gingival inflammations" as used herein comprise all kinds of gingival inflammations such as gingivosis, necrotizing ulcerative gingivitis (including acute and recurrent), diabetic gingivitis, proliferative gingivitis (including leukemic), hormonal gingivitis, plasma cell gingivitis, suppurating gingivitis, fusospirochetal gingivitis, and gingival inflammations caused by pharmaceutical agents such as diphenylhydantoin gingivitis. Also, the term "dental periostitis" as used herein comprises those that result from gingival inflammations and those that result from apical lesions, and the therapeutic, preventive, and improving agents according to the present invention are effective for both types of dental periostitis, and especially effective for those that result from gingival inflammations.

In the present specification, the term "surgical operations" comprise any surgical operations of the jaw and periodontal area including oral surgeries for tooth extractions, treatment of tumors and deformities (for example, jaw deformity), reconstructions, implants, and orthodontic treatment. The therapeutic, preventive, and improving agents according to the present invention are useful for treating, preventing, and improving alveolar bone defects caused by the surgical operations described above. Furthermore, they are useful for accelerating the healing of wounds resulting from, among the surgical operations described above, especially implant operations, surgical removal of tumors, surgical operations for treating jaw deformity, jaw bone operations (for example, operations to treat maxillary or mandibular protrusion), and the like.

The term "alveolar bone defect" as used herein refers to the state in which a part of alveolar bone is defected or lost due to surgical operations, and the like.

Expression of a gene is regulated by transcriptional regulatory factors which bind to the transcriptional regulatory region of the gene. NFκB is a heterodimer mainly consisting of p65 and p50 subunits, which was identified in 1986 as a transcription factor that binds to an enhancer involved in the expression of immunoglobulin κ light chain gene in B cells (Cell 46 (1986) 705-716). The term "NFκB" is used in this field as the generic term for proteins having a structural relationship and evolutionarily-conserved activity as a transcription factor. NFκB binds to various DNA sequences which initiate transcription of genes of cytokines (interleukin (IL)-1, IL-6, IL-8, tumor necrosis factors (TNF), and the like), angiogenic factors (vascular endothelial growth factors (VEGF) and the like), cell adhesion factors (intercellular adhesion molecule (ICAM)-1, vascular cell adhesion molecule (VCAM)-1, and the like), enzymes (cyclooxygenase (COX)-2, nitric oxide synthase (NOS), and the like), and anti-apoptotic factors (bcl-2, survivin, and the like) (Ann Rev Immunol 14 (1996) 649-683; Immunol Today 19 (1998) 80-88; Trends Mol Med 8 (2002) 385-389; Nat Rev Cancer 2 (2002) 301-310).

Binding sequences (binding regions) of NFκB are known from various literatures (see, for example, "Bunshi Saibou Seibutugaku Jiten (Dictionary of Molecular and Cellular Biology)" Tokyo Kagaku Dojin, published in 1997, p. 891). Specific binding sequences include GGGRHTYYHC (R is A or G; Y is C or T; and H is A, C, or T) (SEQ ID NO: 2). For example, gggatttccc (SEQ ID NO: 3) and gggactttcc (SEQ ID NO: 4) may be included; however, the present invention is not limited thereto.

In the present specification, the term "NFκB decoy" indicates a molecule that suppresses the activity of NFκB by binding with the transcription factor NFκB within cells, and thus inhibiting the binding of NFκB with the corresponding binding sequences in the genome. "Decoy" is an English equivalent of "bait", and in this field, a substance having a structure similar to a substance to which a certain substance is supposed to bind or act on is called a "decoy". As decoys of transcription factors which bind to their binding regions on genomic genes, known mainly are double-stranded oligonucleotides having the same nucleotide sequences as that of the binding regions (Patent Document 1, Patent No. 3392143, WO 95/11687). When a decoy comprising such an oligonucleotide coexists, some transcription factor molecules do not bind to the original binding region on the genomic gene, but binds to the oligonucleotide decoy. Therefore, the number of transcription factor molecules which bind to the original binding region on the genomic gene is reduced, and as a result, the activity of the transcription factor is decreased. In such a case, the oligonucleotide is called a decoy, because it functions as a dummy (bait) for the true binding region on the genomic gene and binds the transcription factors. Various oligonucleotide decoys for NFκB are also known, and various pharmacological effects have been confirmed (Japanese Patent Application *Kokai* Publication No. (JP-A) 2005-160464 (unexamined, published Japanese patent application); WO 96/35430; WO 02/066070; WO 03/043663; WO 03/082331; WO 03/099339; WO 04/026342; WO 05/004913; WO 05/004914).

In decoys, generally, other nucleotides are also linked to both ends of the NFκB binding sequences (called binding regions, consensus sequences, or core sequences). These nucleotide portions may be called "additional sequences". The nucleotide portions of each end consist of one or more nucleotides, preferably one to twenty nucleotides, more preferably one to ten nucleotides, and most preferably one to seven nucleotides.

In the present invention, especially preferable decoys include double-stranded oligonucleotides, but are not limited thereto. Although the double strands are preferably completely complementary sequences, they may comprise one or more (preferably one or two) non-complementary nucleotide pairs, as long as they can bind a transcription factor. That is, a typical decoy in the present invention is a double-stranded oligonucleotide comprising a sense strand oligonucleotide having the structure of 5'- end-linked additional sequence - binding sequence - end-linked additional sequence -3' and an antisense strand nucleotide completely complementary to the sense strand oligonucleotide. Herein, the number of binding sequences between both end-linked additional sequences is not limited, and a plurality of binding sequences may be linked in tandem, directly, or with one or few nucleotides in between.

Oligonucleotides constructing the decoy may be DNAs or RNAs, and may comprise one or more modified nucleotides. They comprise, for example, nucleic acids with a backbone modified with, for example, phosphorothioate, methyl phosphoate, phosphorodithioate, phosphoroamidate, boranophosphate, methoxyethyl phosphoate, and morpholinophosphoroamidate; peptide nucleic acids (PNAs); locked nucleic acids (LNAs); and nucleic acids comprising nucleotides that are dinitro-phenylated (DNP) and O-methylated. In the present invention, oligonucleotides may be synthesized with ribonucleosides in place of deoxyribonucleosides to be modified in the oligonucleotide, and the ribonucleosides may be modified. For example, O-methylation, dinitro-phenylation, and the like are usually used for modification of ribonucleosides, and in some cases, the method described above is preferable. Among them, oligonucleotides comprising phosphorothioated nucleotides (that is, the linkage between nucleosides are phosphorothioate linkages) are preferable. Either all nucleotides or one or more nucleotides constituting the oligonucleotide may be modified.

For example, nucleotide strands including double-strands comprising oligonucleotides including the undermentioned nucleotide sequences and their complementary strands may be used as NFκB decoys in the present invention. As described above, in NFκB decoys of the present invention, the undermentioned nucleotide sequences may be linked with other nucleotides. Furthermore, as long as a transcription factor can bind, an NFκB decoy in the present invention may comprise at least one modified nucleotide. Alternatively, as long as a double-stranded structure is maintained, such as by a hairpin structure, the nucleotide strand may be a single strand.
5'-CCTTGAAGGGATTTCCCTCC-3' (SEQ ID NO: 5)
5'-AGTTGAGGACTTTCCAGGC-3' (SEQ ID NO: 7)
5'-AGTTGAGGGGACTTTCCCAGGC-3' (SEQ ID NO: 9)

Examples of preferable NFκB decoys used in the present invention include:
a decoy comprising a complementary double-stranded oligonucleotide of
   5'-CCTTGAAGGGATTTCCCTCC-3' (SEQ ID NO: 5) and
   3'-GGAACTTCCCTAAAGGGAGG-5' (SEQ ID NO: 6);
a decoy comprising a complementary double-stranded oligonucleotide of
   5'-AGTTGAGGACTTTCCAGGC-3' (SEQ ID NO: 7) and
   3'-TCAACTCCTGAAAGGTCCG-5' (SEQ ID NO: 8); and
a decoy comprising a complementary double-stranded oligonucleotide of
   5'-AGTTGAGGGGACTTTCCCAGGC-3' (SEQ ID NO: 9) and
   3'-TCAACTCCCCTGAAAGGGTCCG-5' (SEQ ID NO: 10).

However, decoys of the present invention are not limited to those composed of double-stranded oligonucleotides. For example, as long as NFκB binds to the double-stranded part formed by a covalent bond between the NFκB binding sequence and its complementary sequence and transcription activation by NFκB is suppressed thereby, decoys other than double-stranded decoys may be used as decoys of the present invention. Such decoys include dumbbell-type decoys (also called ribbon-type or staple-type) which is a cyclic single-stranded oligonucleotide strand having both a NFκB binding sequence and its complementary sequence where these sequences form a double-strand by intramolecular binding; hairpin-type decoys comprising a non-cyclic single-stranded oligonucleotide; and the like.

Agents of the present invention comprise one or more NFκB decoys as an active ingredient. Decoys included in the agents are not limited to one kind, as long as the decoys do not inhibit each other's actions.

Decoys used in the present invention can be manufactured with known nucleic acid synthesizing methods. For example, various commonly used methods such as the phosphoamidide method (Am J Chem Soc 103 (1981) 3185-3191) and the phosphite triester method (Nature 310 (1984) 105-111) are known. Also, where appropriate, DNA synthesizers and the like may be used.

Whether or not an oligonucleotide can function as a decoy by binding to transcription factors can be verified through known binding activity tests. In testing binding activity for NFκB, for example, TransAM NFκB p65 Transcription Factor Assay Kit (ACTIVE MOTIF) may be used. The tests can be easily carried out based on documents appended to the Kit, or by protocol modifications one skilled in the art would routinely perform. That is, those skilled in the art can easily check whether oligonucleotides produced by arbitrarily modifying (for example, by substituting, adding, inserting, deleting, or modifying nucleotides constituting the decoys) known NFκB decoys, or decoys shown in the present specification with specified sequences will act as decoys or not. Accordingly, also from such a view point, NFκB decoys used in the present invention are not limited to, for example, oligonucleotides that were verified to have activity in the Examples.

As shown in the Examples, the present inventors carried out experiments using the alveolar pyorrhea model and the bone defect model and demonstrated that, in bone metabolism mechanisms, NFκB decoys suppress excessive inflammation reactions and bone resorption, promote new bone formation, and prompt the early restoration mechanism. Since such effects of NFκB decoys in the process of healing alveolar bone defects are considered to be ascribable to the effect of suppressing transcription activation by NFκB, materials other than decoys which suppress transcription activation by NFκB are considered to bring effects similar to those of the agents of the present invention. As another embodiment, the present invention provides methods and medicines for treating, preventing, or improving periodontal diseases, diseases accompanied by alveolar bone defects caused by surgical operations, and the like, comprising without limitation known ingredients such as antisenses, ribozymes, aptamers, and siRNAs, which are known to regulate transcription activity in cells. Methods for manufacturing antisenses, ribozymes, aptamers, and siRNAs against transcription factors with known binding nucleotide sequences are known in the art. Accordingly, those skilled in the art can easily prepare, based on known techniques, suitable antisenses, ribozymes, aptamers, and siRNAs for various NFκBs with known binding sequences.

The present invention relates to agents for treating, preventing, or improving alveolar bone defects caused by periodontal diseases and surgical operations, which comprise NFκB decoys as an active ingredient. In the present specification, "treatment" includes not only complete healing of alveolar bone defects and the accompanying symptoms caused by periodontal diseases and surgical operations, but also improvement of at least a part of those symptoms. Also, "prevention" refers to suppression of at least a part of alveolar bone defects and accompanying symptoms caused by periodontal diseases and surgical operations by administering the agent before onset of periodontal diseases, or before or immediately after surgical operations. Furthermore, "prevention" comprises administration after one treatment to prevent recurrence. Moreover, "improvement" indicates relieving of at least a part of alveolar bone defects and accompanying symptoms caused by periodontal diseases and surgical operations.

Therapeutic, preventive, and improving agents of the present invention can be administered, for example, by injecting, applying, or implanting the agents. Therefore, the therapeutic, preventive, and improving agents of the present invention can be formulated as injections, ointments, or implanting agents to be administered through application or implantation. As agents for treating bone defects, injections or implanting agents are preferable. However, the present invention is not limited thereto, and any dosage form and administration method can be adopted, as long as they enable topical administration to periodontal tissue. They can be arbitrarily selected by those skilled in the art.

As a composition for an injection, NFκB decoys dissolved into a pharmaceutically acceptable carrier (liquid) may be used. Examples of carriers include phosphate buffered saline (PBS) and collagen solutions. However, the present invention is not limited to these carriers and any known carriers can be used as needed.

As a composition for an ointment, NFκB decoys mixed with a pharmaceutically acceptable base material, such as, but not limited thereto, polyethylene glycol and glycerin, can be used.

NFκB decoys attached to or mixed with a known base material suitable for an implanting agent, such as, but not to limited to, collagen, gelatin, and hydroxyapatite, can be administered by implanting into the affected area as the implanting agent.

When the agent of the present invention is used for treating or improving periodontal diseases, especially gingival inflammations and alveolar pyorrhea, and if there is a periodontal pocket between tooth and gum (gingiva), the agent of the present invention may be injected into the pocket. In such a case, ointments or implanting agents are preferable as the dosage form. Alternatively, injections for topical administration to the gingival site are also a preferable dosage form in the present invention.

Whichever dosage form is adopted, base materials and carriers that can gradually release the NFκB decoy contained in the agent are preferable. Those skilled in the art can select suitable base materials and carriers from those used in the art. For example, gelatin and collagen are preferable. Other suitable carriers include, for example, AteloGene™ Local Use (KOKEN), but are not limited thereto. That is, a pharmaceutical composition comprising an NFκB decoy and a nucleic acid transfection agent is preferable as a therapeutic, preventive, and improving agent of the present invention. More specifically, a pharmaceutical composition comprising an NFκB decoy and a nucleic acid transfection agent to be administered by injection into gingiva is preferable as a therapeutic, preventive, and improving agent of the present invention. Nucleic acid transfection agents comprise gelatin, collagen, atelocollagen, and the like. Alternatively, the present invention relates to the use of NFκB decoys in manufacturing a pharmaceutical composition for treating either or both of periodontal diseases and alveolar bone defects. Also, the present invention provides the use of NFκB decoys in treating either or both of periodontal diseases and alveolar bone defects. Various excipients, stabilizers, lubricants, additives, and the like can be added to the improving agents of the present invention where appropriate.

Atelocollagen is obtained by removing telopeptide from collagen. Telopeptide can be removed from collagen by treating collagen with proteolytic enzymes such as pepsin. Since telopeptides have strong antigenicity, their removal results in a safer collagen. By mixing atelocollagen with, for example, a nucleotide strand (that is, an NFκB decoy), a complex comprising both can be obtained. Atelocollagen for complex formation may be granular or fibrous. The size of granular atelocollagen may be, for example, between 300 nm and 300 mcm, or between 300 nm and 30 mcm. A complex is formed by mixing with nucleotide strands in a suitable buffer solution at a temperature that does not decompose collagen. The shape and size of the complex may be regulated by conditions such as collagen concentration, salt concentration, and temperature. The ratio between collagen and nucleotide strand may be suitably selected, for example, from the range between 1:1 and 1:100.

Agents comprising decoys of the present invention as an active ingredient may be used for treating, preventing, or improving alveolar bone defects in humans and other mammals. That is, the present invention provides methods for either or both of treating and improving periodontal diseases comprising the step of administering NFκB decoys to subjects with either or both of periodontal diseases and alveolar bone defects caused by surgical operations. In the present invention, NFκB decoys are administered into gingiva preferably by injection. NFκB decoys may be administered together with a nucleic acid transfection agent. Alternatively, the present invention provides methods for either or both of treating and improving alveolar bone defects comprising the step of administering NFκB decoys to the alveolar bone defect site. In the present invention, NFκB decoys are preferably administered by implanting into the defect site to be treated. NFκB decoys may be administered as a composition with a nucleic acid transfection agent. Nucleic acid transfection agents such as gelatin, collagen, or atelocollagen may be combined in the composition to be administered by implantation. Administration by implantation refers to topically administering to the defect site and retaining therein a pharmaceutical composition to be administered. A pharmaceutical composition may be surgically administered to the defect site, or may be administered by implantation into the defect site by injection.

The amount of NFκB decoys comprised in the agent to be administrated is different according to the conditions of the subjects to be administered, such as age, weight, symptoms, administration methods, and routes. Those skilled in the art can determine the suitable dose taking account of these conditions. For example, 0.05 to 1000 mg, preferably 0.1 to 100 mg for an adult (60 kg) per day can be administered once or divided into several doses. For subjects other than humans, those skilled in the art can determine the suitable dose taking account of the weight, severity of symptoms, and the like.

The agents of the present invention can comprise, in addition to NFκB decoys and as long as the effect of NFκB decoys is not inhibited, other active ingredients which can be used for periodontal diseases and in surgical operations accompanied by alveolar bone defects. Alternatively, the agents of the present invention can be used in combination with other formulations comprising such active ingredients by co-administration or administration with a time difference. Ingredients which may be used in combination with the agents of the present invention include antibiotics, hemostatic agents, and the like. The antibiotics include, for example, penicillin, erythromycin, and tetracycline. Use in combination with tetracycline is especially preferable. The hemostatic agents include tranexamic acid. The dose regimen and the dose of the antibiotics and hemostatic agents are well known to those skilled in the art, and when using in combination with the agents of the present invention, the suitable dose and the like can be determined based on such well known information.

The present invention will be illustrated in the following examples, but it is not limited to these descriptions.

### [Examples]

As periodontal disease models, two kinds of animal models were used in the following Examples. The first is an alveolar pyorrhea model, a model produced by cotton thread ligation at the gingival crevice using beagle dogs (herein, also called the "thread model") in which deposition of dental plaque induces gingival inflammations and causes resorption (regression) of alveolar bone. In order to prepare such a model, according to the method described in the Journal of the Japanese Society of Periodontology (Nihon Shishubyo Gakkai Kaishi), 36 (4) (1994) 823-833, a thread (silk thread No. 3) was ligated at gingival crevices of each of the maxillary and mandibular, right and left, lateral incisors. Silk thread ligation at gingival crevices will cause stagnation of the self-cleaning action within the oral cavity, and deposition of dental plaque induces gingival inflammations, causing resorption (regression) of alveolar bone.

The other model is a model of bone defect caused by apical lesions or surgical operations (herein, also called the "bone defect model"). In order to prepare such a model, according to the method described in J. Periodont. Res. 38 (2003) 97-103, under intravenous anesthesia, alveolar bone corresponding to root furcations of right and left, mandibular premolars were defected with a bar with a dental engine.

In the present Examples, a double-stranded decoy which comprises an oligonucleotide comprising the sequence of CCTTGAAGGGATTTCCCTCC (SEQ ID NO: 1) as NFκB decoy and an oligonucleotide comprising a sequence completely complementary to this sequence, and in which all of linkages between nucleotides are modified with phosphorothioate was used. Also, as a scrambled decoy, a double-stranded decoy which comprises two strands of oligonucleotides comprising the sequence of CATGTCGTCACTGCGCTCAT (SEQ ID NO: 11) and a sequence completely complementary to this sequence, and in which all of linkages between nucleotides are modified with phosphorothioate was used. These double-stranded decoys comprising such sequences are known not to interfere with NFκB activity when introduced into cells (see, for example, Patent Documents 1-3).

### (1) Effects of NFκB decoy oligonucleotide on periodontal diseases in thread models

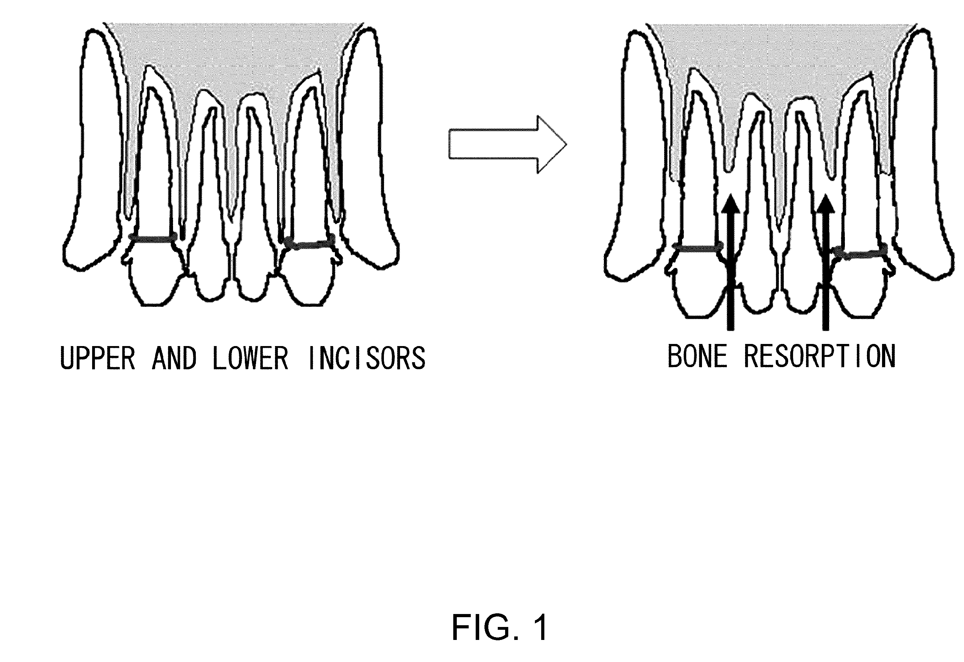

A total of 12 (four each for the one month model, two month model, and three month model) beagle dogs (male, 10-12 months old) were used. The onset mechanism of periodontal diseases is as follows: deposition of food and dental plaque into periodontal pockets induces infections which progress into periodontal diseases; this, results in inflammations that cause resorption (regression) of alveolar bone. Therefore, wrapping thread as in the present model increases food and dental plaque deposition into periodontal pockets and causes periodontal diseases. Fig. 1 shows schematic diagrams of the mechanism. The left side of Fig. 1 schematically shows the appearance at the time the model is prepared by ligating thread, and as time advances, resorption (regression) of alveolar bone occurs as indicated on the right side.

After preparation of the models, the NFκB decoy was administered into root gingival sites of left, maxillary and mandibular, lateral incisors, and the scrambled decoy (control) was administered into root gingival sites of right, maxillary and mandibular, lateral incisors, at a dosage of 1 mg/site, every two weeks, by injection. The decoys were prepared by mixing the decoy solution (dissolved in TE buffer) with AteloGene^{™} of AteloGene^{™} Local Use (KOKEN) Kit at a volume ratio of 1:1, and making adjustments so that the final decoy concentration became 1 mg/100 µL. These prepared decoys were then used.

Samples from four dogs were prepared one month, two months, and three months after model preparation, respectively. The dental root lengths were measured in three ways, that is, direct measurement of exposed dental root length in samples isolated after dissection, evaluation using X-ray photographs taken throughout the feeding and observation period, and bone density measurement with DEXA (Dual Energy X-ray absorptiometry; X-ray bone density measuring apparatus).

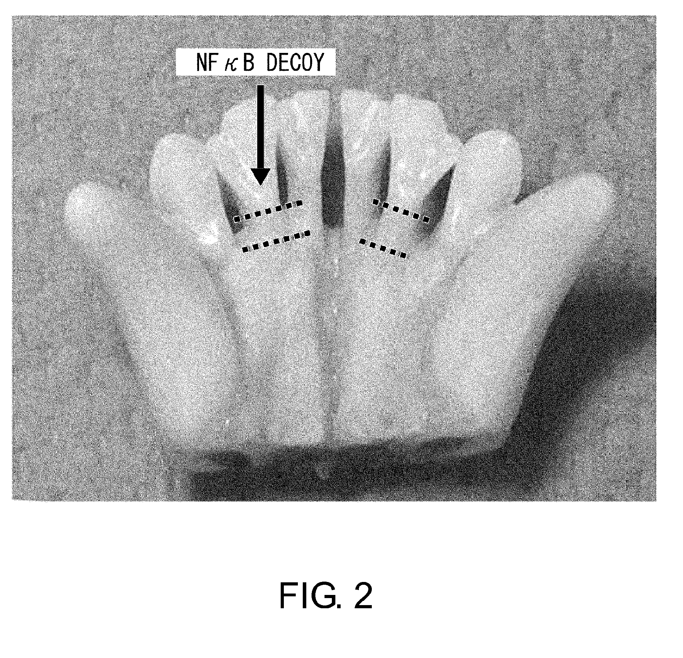

The photograph of Fig. 2 presents an isolated sample (mandible) obtained one month after model preparation, in which attached soft tissue was removed with sodium hypochlorite to make the exposed area of the dental roots more visible. When dental root lengths of mandibular, right and left, lateral incisors were measured and compared, in the right lateral incisor site where the scrambled decoy was administered, regression of alveolar bone was found (exposed dental root: the part between two dashed lines), while in the alveolar bone of the left lateral incisor where the NFκB decoy was administered, bone resorption (regression) was suppressed.

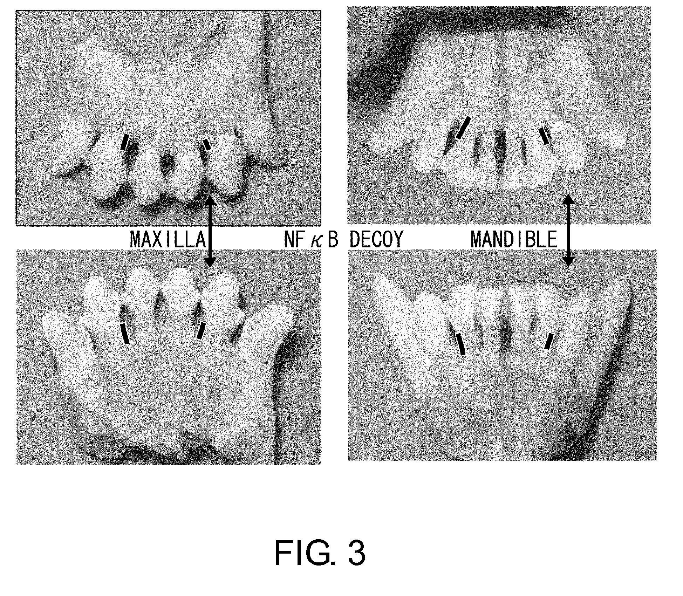

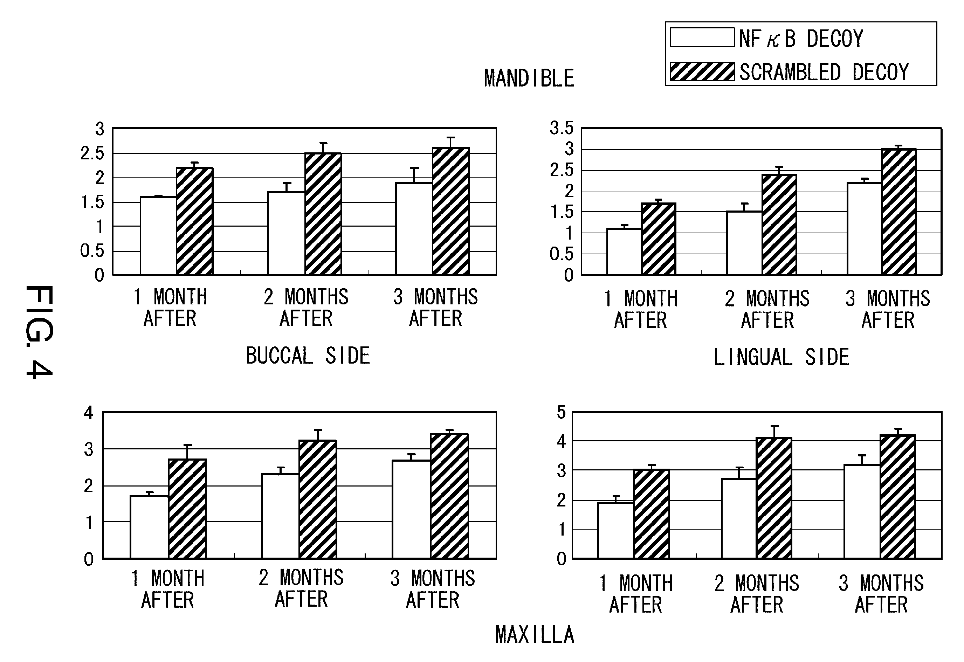

In addition, dental root lengths of maxillary and mandibular, right and left, lateral incisors were similarly measured one month after model preparation (Fig. 3). The measured sites were the mesial side of maxillary lateral incisors and the distal side of mandibular lateral incisors. In both maxilla and mandible, alveolar bone (in Fig. 3, indicated by arrows) of left lateral incisors where the NFκB decoy was administered was conserved and exposure of the dental root (in Fig. 3, the black lines) was suppressed, while regression of alveolar bone of right lateral incisors where the scrambled decoy was administered progressed and exposure of the dental root (black lines) was significant. Fig. 4 presents graphs showing exposed dental root lengths of maxillary and mandibular, right and left, lateral incisors one month, two months, and three months after model preparation. As a whole, the exposed length became larger as time progressed, and smaller length shows suppression of bone resorption and exposure. In all of the one month models and two month models, a difference between the exposed dental root lengths was found between NFκB decoy administration and scrambled decoy administration. As a result, it was shown that in the NFκB decoy-administered group, exposure is suppressed.

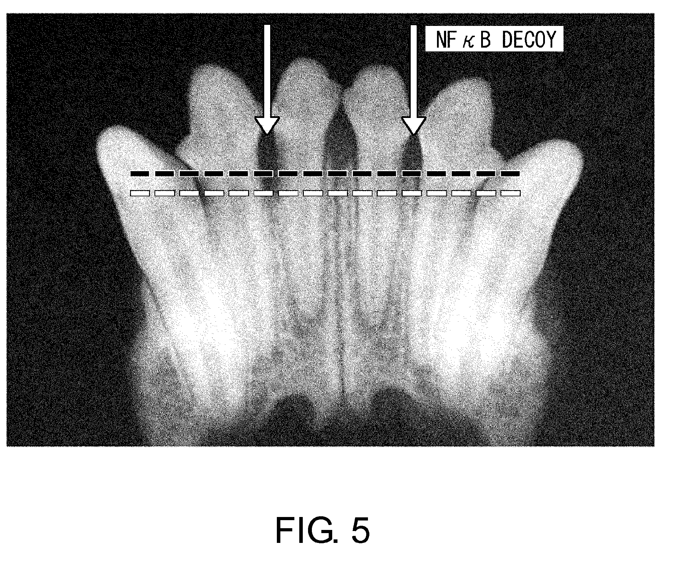

Also in the X-ray picture of the maxilla taken during dissection two months after model preparation (Fig. 5), it was revealed that bone resorption (regression) is suppressed in the tip (black line) of the alveolar bone (indicated by the right side arrow) of maxillary left lateral incisor where the NFκB decoy was administered than the tip (white line) of the alveolar bone (indicated by the left side arrow) of right lateral incisor where the scrambled decoy was administered.

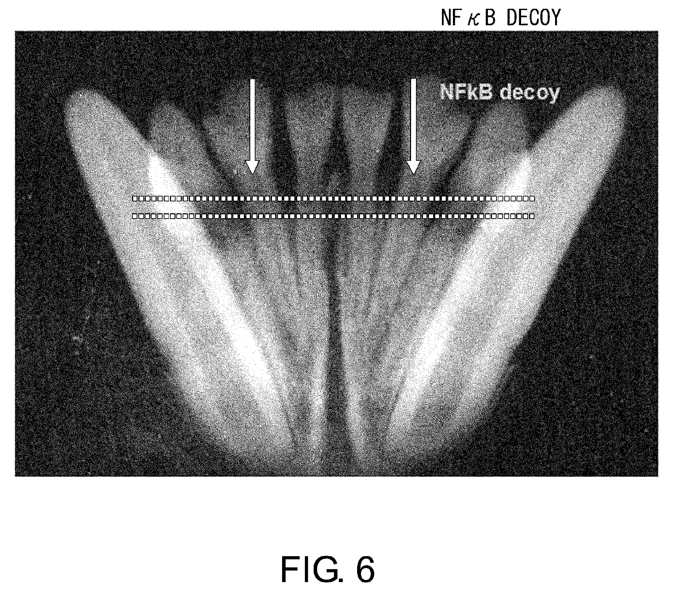

Similarly, in the X-ray picture of the mandible taken during dissection two months after model preparation (Fig. 6), as same as in maxilla, it was confirmed that bone resorption (regression) of the alveolar bone of left lateral incisor (indicated by the right side arrow) where the NFκB decoy was administered was suppressed than in the right lateral incisor site (indicated by the left side arrow) where the scrambled decoy was administered.

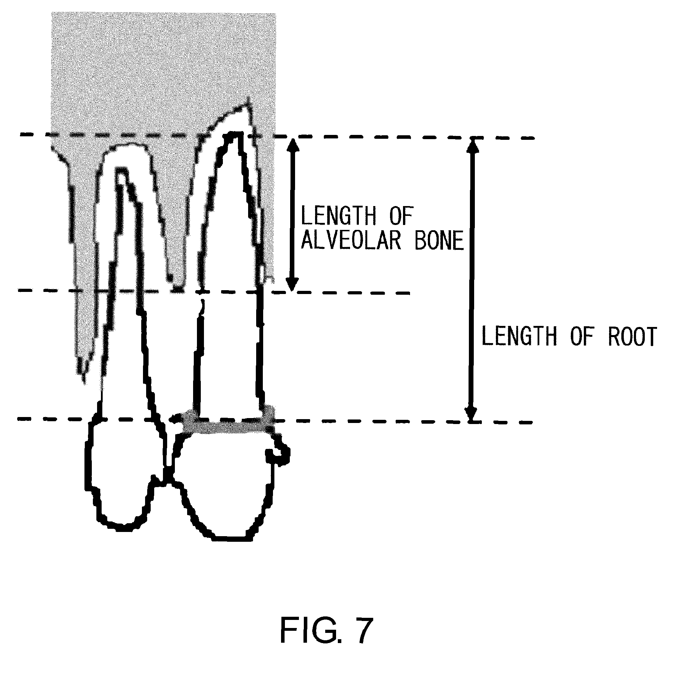

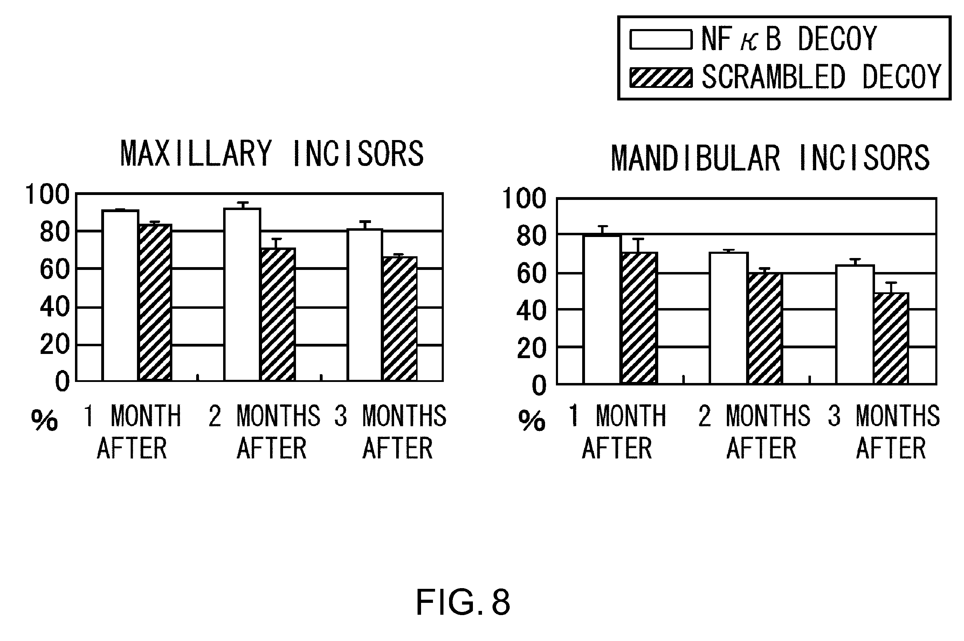

Next, resorptions of the alveolar bone were quantified, and the effects of the NFκB decoy and scrambled decoy was compared. When X-ray pictures are taken, the images expand or contract depending on the angle, thus measured absolute values can not be used for comparison with time. Therefore, as the control, dental root length with no length change was used (see Fig. 7). More specifically, by dividing the length of alveolar bone with the length of dental root from the neck of tooth, "length of alveolar bone/length of dental root ratio" was obtained. The length of alveolar bone/length of dental root ratios measured one month, two months, and three months after model preparation are called as "length of remaining alveolar bone/length of dental root ratios". As the breeding period becomes longer, resorption (regression) of the alveolar bone progresses naturally, and the length of remaining alveolar bone/length of dental root ratio becomes smaller. However, it was shown that the progress of bone resorption over time in NFκB decoy administered group was suppressed compared with that in scrambled decoy administered group, and that the NFκB decoy contributes to conserve alveolar bone (Fig. 8).

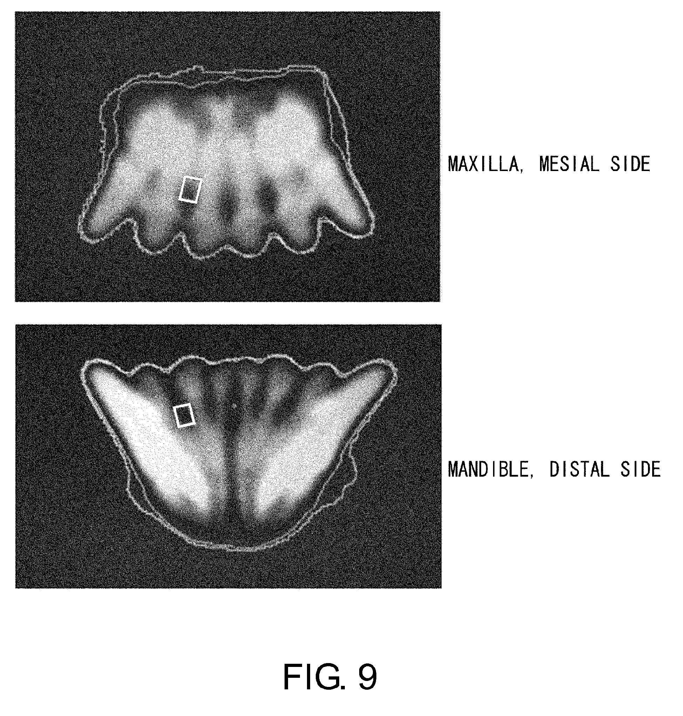

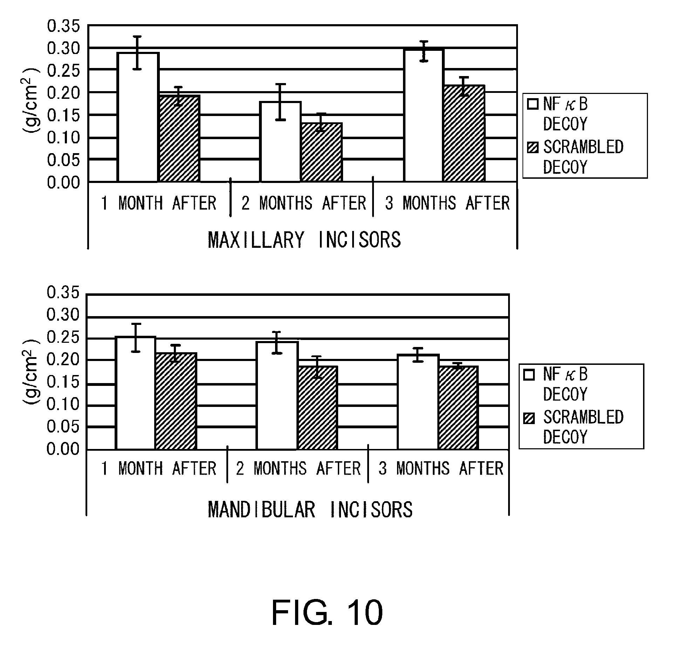

Further, as another evaluation method of alveolar bone resorption, DEXA (Dual Energy X-ray absorptiometry; X-ray bone density measuring apparatus) analysis was performed. The analysis images are shown in Fig. 9. The measured sites (parts surrounded by white rectangles) were the mesial side of maxillary lateral incisor and the distal side of mandibular lateral incisor. The results one month, two months, and three months after model preparation are shown in Fig. 10. The bone density of alveolar bone in NFκB decoy administered group was elevated compared with that in scrambled decoy administered group, and it was recognized that the NFκB decoy suppresses resorption (regression) of alveolar bone and has a bone trabeculae conserving effect.

Furthermore, in these thread-ligated model dogs, IL-6 in gingival crevicular fluid was measured two months after model preparation. IL-6 is a cytokine that elicits inflammation, and it is well known to induce differentiation of osteoclasts and trigger bone destruction. That is, the amount of IL-6 in gingival crevicular fluid is an indicator of inflammation, as well as a predictive factor of bone destruction.

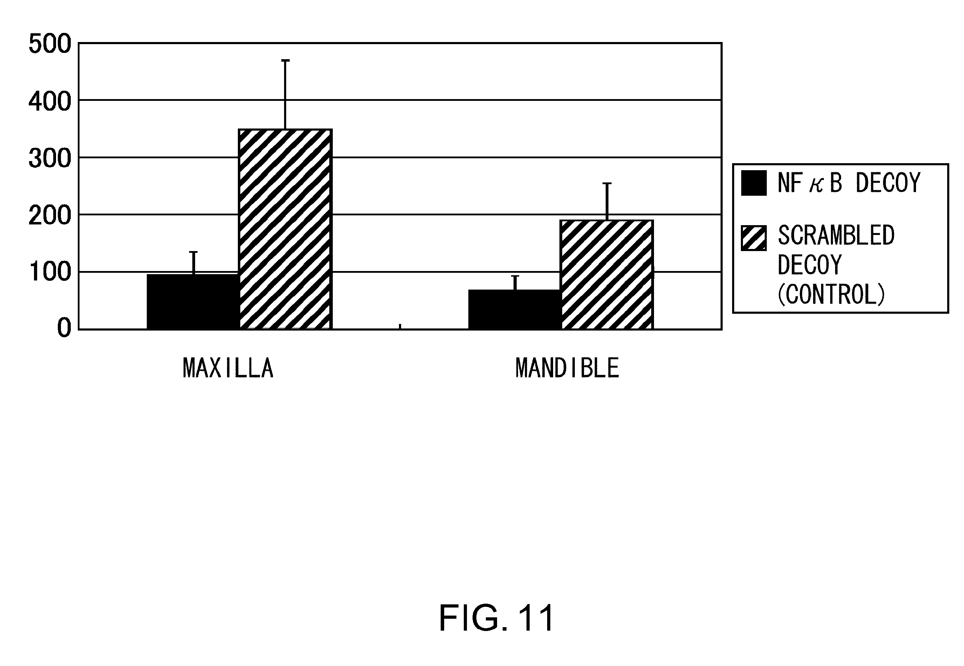

IL-6 was measured with ELISA according to conventional means. The results are shown in Table 1 and Fig. 11.

**Table 1**

| IL-6(pg/ml) | NFκB decoy | Scrambled decoy (control) |
|---|---|---|
| Maxilla | 95.83 | 348.96 |
| Mandible | 68.67 | 190.74 |

In both the maxilla and mandible, IL-6 in gingival crevicular fluid was reduced in the NFκB decoy administered group compared to the scrambled decoy administered group, which is the control group. Also from this result, it was discovered that NFκB decoy suppresses inflammation in periodontal diseases and suppresses bone destruction.

All prior art documents cited in the present specification are incorporated herein by reference.

### (2) Effects of NFκB decoy oligonucleotide in periodontal disease-caused bone defect model

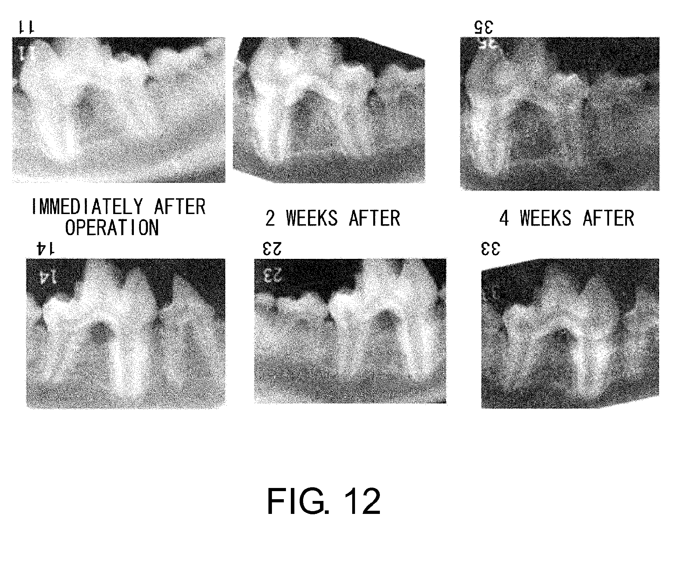

The beagle dog (male, 10-12 months old) bone defect models used in the present Examples were prepared as follows: under intravenous anesthesia, in both right and left, gingival flaps were prepared from mesial parts of premolars to expose alveolar bones, and three-wall bone defects with a diameter of 5 mm were made with a dental bar at root furcation sites of molars. For the left molar bone defect site, NFκB decoy mixed with a collagen base material (AteloGene^{™}), and for the right molar bone defect site, scrambled decoy mixed in the same way, was administered by implantation, respectively. The decoys used were adjusted to be 1 mg/100 µL as described in the above Example, and used. After administration by implantation of decoys, the gingival flaps were stitched together, and restored. The gingival flap sites healed two weeks after operation, and infection stimulations from outside and the like were blocked. The bone defect restoring process was observed between immediately after operation and four weeks after operation by taking X-ray pictures at two week intervals (Fig. 12).

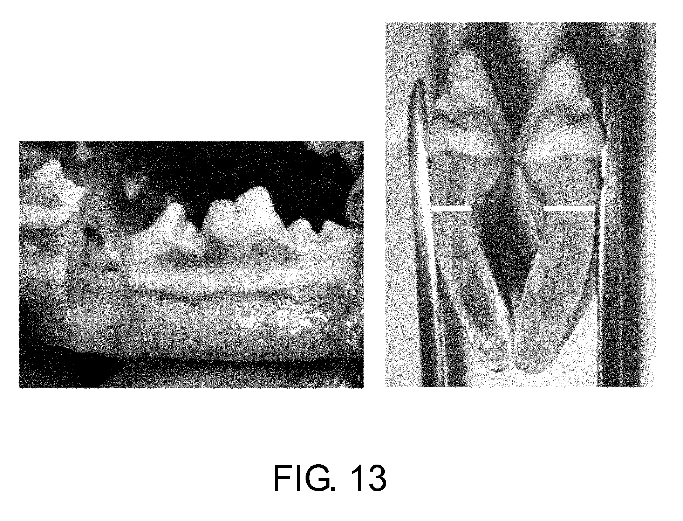

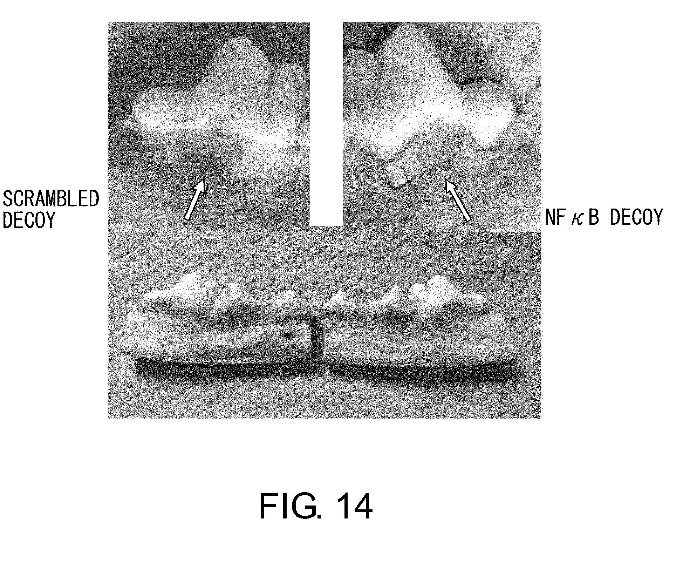

One month after operation, jaw bones were taken out, and their cross-sectional planes were observed. It was verified that thickness of right cortical bones where the NFκB decoy was administered had increased (Fig. 13). In addition, when the bone defect sites of the models one month after operation were compared, in the NFκB decoy administered sites, complete cortical bones were formed on the surface of defect sites, while in the scrambled decoy administered sites, only soft and fragile cortical bones were observed (Fig. 14).

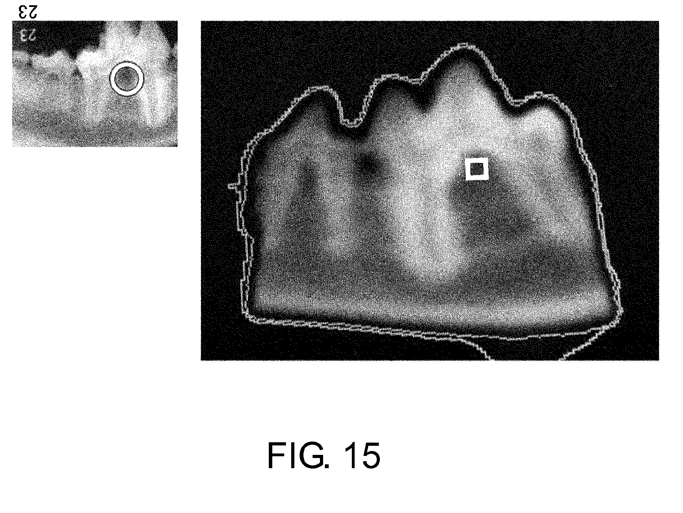

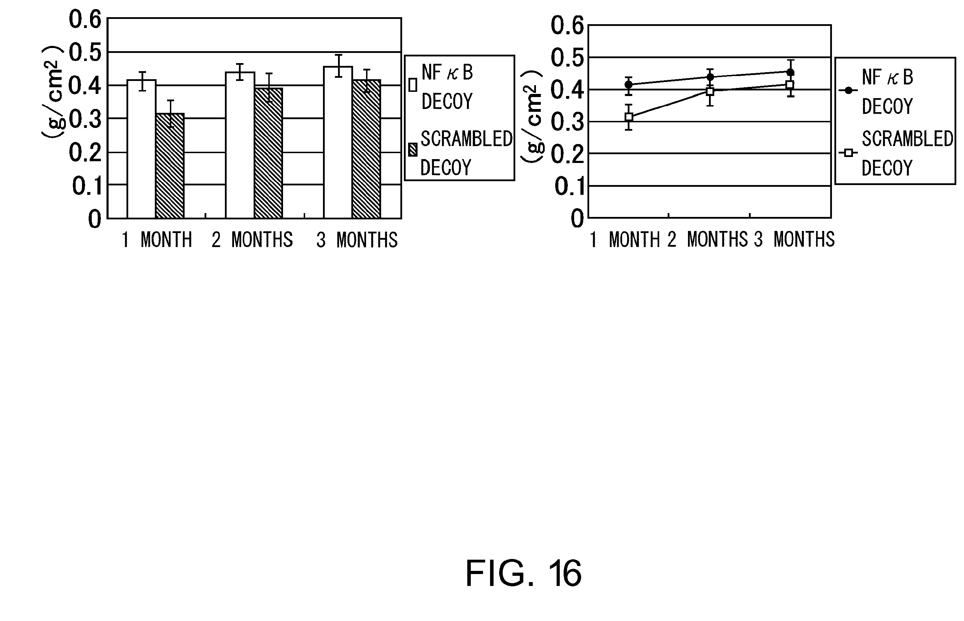

In order to numerically evaluate the restoration of these bone defect sites, bone density measurement with DEXA was performed. Bone defect sites identified by dental X-ray (in the left side photograph of Fig. 15, the part surrounded by a circle) were analyzed with DEXA. The analyzed image is shown in the right side photograph of Fig. 15. The analysis was performed for model animals one month, two months, and three months after operation. In both of the NFκB decoy administered sites and the scrambled decoy administered sites, bone density increased with time due to the healing mechanism; however there was difference in restoration rate between the two groups. The NFκB decoy administered sites healed about one month faster than the scrambled decoy administered sites (Fig. 16).

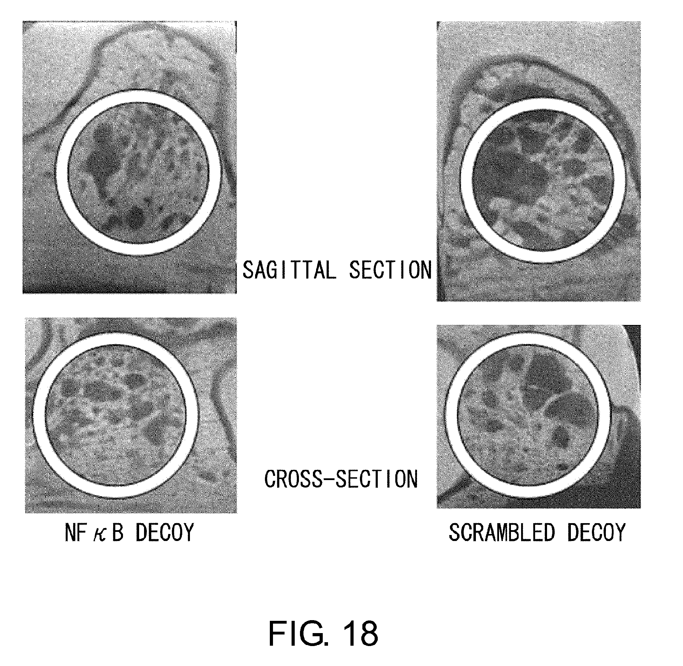

Furthermore, restoration of bone defect sites was analyzed by using an X-ray micro CT (for small animals: SHIMADZU Kyoto Japan, X-ray CT system SMX-100CT-SV) and an analysis software (X-ray Image Viewer Version 4.0). In this analysis, images, as well as trabeculae occupancy per unit volume, which corresponds to bone density, and the like can be measured. Analysis was performed one month, two months, and three months after operation. Sites corresponding to bone defects identified by X-ray pictures were subjected to X-ray micro CT, and CT images were obtained. Images one month after operation are shown in Fig. 17. In Fig. 17, parts surrounded by circles are the spongy bones, and the area surrounded by each circle is identical. In the NFκB decoy administered side, neonatal bones appear white in the bone defect site, while in the scrambled decoy administered side, bone defect sites appear black since restoration is delayed and trabeculae is sparse. Images two months after operation are shown in Fig. 18. In the NFκB decoy administered side, the proportion of neonatal bones occupying the parts surrounded by circle increased, and the restoration had reached almost the same level as that of normal spongy bone. In the scrambled decoy administered side, restoration of bone defect sites is delayed, and the proportion of cavity which appears black (unrestored parts) is large.

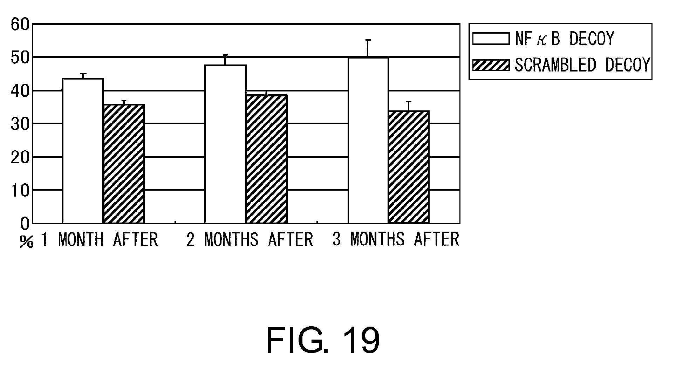

In order to numerically evaluate these analysis results, the proportion of neonatal bones occupying the bone defect sites were determined as volume ratios of trabeculae/measured space, and obtained values were plotted in a graph (Fig. 19). In the NFκB decoy administered side, bone amount increased with time and reached almost a plateau two months after operation, while in the scrambled decoy administered side, a significant improvement in the healing process was not observed.

### Industrial Applicability

Experiments using the alveolar pyorrhea model and the bone defect model prepared by the present inventors demonstrated that NFκB decoys suppress excessive inflammatory reactions and bone resorption in the bone metabolism mechanism, promotes neonatal bone formation and early restoration, by suppressing transcription activation by NFκB. Based on such effects of NFκB decoys in the healing process of alveolar bone defects, the present invention provides novel means for treating, preventing, or improving periodontal diseases, and diseases accompanying alveolar bone defects caused by surgical operations, and the like.

## Claims

1. An agent for treating, preventing, or improving either or both of a periodontal disease and a surgical operation-induced alveolar bone defect, comprising a NFκB decoy as an active ingredient.

2. The therapeutic, preventive, or improving agent according to claim 1, wherein the periodontal disease is selected from the group consisting of periodontal infection, gingival inflammation, dental periostitis, and apical lesion arising from dental caries.

3. The therapeutic, preventive, or improving agent according to claim 1, which is an injection.

4. The therapeutic, preventive, or improving agent according to claim 1, which is in a dosage form in which the NFκB decoy is attached to a collagen base material.

5. The therapeutic, preventive, or improving agent according to claim 1, wherein at least one of the linkages between nucleotides comprised in the NFκB decoy is a phosphorothioate linkage.

6. The therapeutic, preventive, or improving agent according to claim 5, wherein all of the linkages between nucleotides comprised in the NFκB decoy are phosphorothioate linkages.

7. The therapeutic, preventive, or improving agent according to claim 1, wherein the NFκB decoy is a double-stranded oligonucleotide which is a double strand formed by an oligonucleotide comprising CCTTGAAGGGATTTCCCTCC (SEQ ID NO: 1) and an oligonucleotide comprising a sequence completely complementary thereto.

8. A method for treating, preventing, or improving either or both of a periodontal disease and a surgical operation-induced alveolar bone defect, comprising the step of administering an NFκB decoy to a subject affected by either or both of a periodontal disease and a surgical operation-induced alveolar bone defect.

9. The method according to claim 8, wherein the periodontal disease is selected from the group consisting of periodontal infection, gingival inflammation, dental periostitis, and apical lesion arising from dental caries.

10. The method according to claim 8, wherein the NFκB decoy is administered by injection.

11. The method according to claim 8, which comprises a step of administering a NFκB decoy attached to a collagen base material.

12. The method according to claim 8, wherein at least one of the linkages between nucleotides comprised in the NFκB decoy is a phosphorothioate linkage.

13. The method according to claim 12, wherein all of the linkages between nucleotides comprised in the NFκB decoy are phosphorothioate linkages.

14. The method according to claim 8, wherein the NFκB decoy is a double-stranded oligonucleotide which is a double strand formed by an oligonucleotide comprising CCTTGAAGGGATTTCCCTCC (SEQ ID NO: 1) and an oligonucleotide comprising a sequence completely complementary thereto.

15. Use of an NFκB decoy for the manufacture of a pharmaceutical agent for treating, preventing, or improving either or both of a periodontal disease and a surgical operation-induced alveolar bone defect.

16. The use according to claim 15, wherein the periodontal disease is selected from the group consisting of periodontal infection, gingival inflammation, dental periostitis, and apical lesion arising from dental caries.

17. The use according to claim 15, which is an injection.

18. The use according to claim 15, which is in a dosage form in which NFκB decoy is attached to a collagen base material.

19. The use according to claim 15, wherein at least one of the linkages between nucleotides comprised in the NFκB decoy is a phosphorothioate linkage.

20. The use according to claim 19, wherein all of the linkages between nucleotides comprised in the NFκB decoy are phosphorothioate linkages.

21. The use according to claim 15, wherein the NFκB decoy is a double-stranded oligonucleotide which is a double strand formed by an oligonucleotide comprising CCTTGAAGGGATTTCCCTCC (SEQ ID NO: 1) and an oligonucleotide comprising a sequence completely complementary thereto.
